# FASCICULE DE BREVET EUROPEEN

(11) **EP 3 126 842 B1**
(45) Date de publication et mention de la délivrance du brevet: **02.06.2021**
(21) Numéro de dépôt: 15719800.3
(22) Date de dépôt: 03.04.2015
(51) Int. Cl.: C12M 1/00, G01N 33/569, C12Q 1/06, C12Q 1/24

(54) **MÉTHODE D'ANALYSE MICROBIOLOGIQUE D'UN ÉCHANTILLON DANS UN CONTENEUR UNIQUE**
VERFAHREN ZUR MIKROBIOLOGISCHEN ANALYSE EINER PROBE IN EINEM EINZIGARTIGEN BEHÄLTER
METHOD OF MICROBIOLOGICAL ANALYSIS OF A SAMPLE IN A UNIQUE CONTAINER

(30) Priorité: 04.04.2014 FR 1453017
(43) Date de publication de la demande: 08.02.2017
(73) Titulaire: Prestodiag, 75013 Paris (FR); Commissariat à l'Énergie Atomique et aux Énergies Alternatives, 75015 Paris (FR); Centre National de la Recherche Scientifique (C.N.R.S.), 75016 Paris (FR)
(72) Inventeur: CALEMCZUK, Roberto, F-38000 Grenoble (FR); CARRARA, David, F-91300 Massy (FR); LIVACHE, Thierry, F-38560 Jarrie (FR); MERCEY, Thibaut, F-75013 Paris (FR); PIAT, Félix, F-22000 Saint Brieuc (FR); ROUPIOZ, Yoann, F-38570 Goncelin (FR); SLIMANI, Sami, F-69200 Venissieux (FR)
(74) Mandataire: Plasseraud IP
(86) Numéro de dépôt international: PCT/FR2015/050875
(87) Numéro de publication internationale: WO 2015/150714

(56) Documents cités:
- WO-A1-2010/017519
- WO-A1-2010/099486
- WO-A1-2010/106282
- WO-A1-2012/004540
- WO-A1-2012/085470
- CN-A- 102 899 242
- US-A- 5 266 486
- US-A- 5 518 923
- US-A1- 2004 009 572
- US-A1- 2008 153 153
- US-A1- 2010 124 763
- US-A1- 2010 216 183
- US-A1- 2011 217 767
- US-A1- 2013 017 131

## Description

La présente invention concerne une méthode d'analyse comprenant la préparation et l'analyse d'un échantillon dans un conteneur souple sans manipulation directe de l'échantillon et sans réouverture du conteneur souple à l'issue de la préparation de l'échantillon, ainsi qu'un conteneur et un kit permettant la mise en œuvre de cette méthode.

La sécurité sanitaire est un enjeu sociétal important. Elle est en partie assurée par des contrôles microbiologiques réguliers, normalisés et souvent obligatoires. La sécurité sanitaire concerne divers domaines comme l'agroalimentaire, l'environnement ou la santé. En agroalimentaire par exemple, les aliments et leur environnement sont soumis à des contrôles microbiologiques à différents stades de la production. Parmi les sources majeures d'intoxications alimentaires, nous pouvons citer à titre d'exemples de microorganismes pathogènes les *Salmonella*, *Listeria*, ainsi que les souches entéro-hémorragiques d'*Escherichia coli.*

L'objectif des analyses menées, notamment dans l'industrie agro-alimentaire, est d'obtenir le plus rapidement possible et en toute sécurité les informations liées à la présence/absence et/ou à la quantification de pathogènes dans un échantillon.

Il existe des méthodes d'analyse « génériques » qui permettent de détecter la présence/absence de microorganismes dans un échantillon, sans distinction de la nature du contaminant microbien (voir par exemple US 5,795,773 ou WO 2010/017519). Il s'agit dans ce cas de détecter la stérilité ou l'absence de stérilité d'un échantillon. Ce type d'analyse générique est utilisé dans l'industrie agroalimentaire, par exemple pour les analyses sur le lait UHT, et est également couramment utilisé dans l'analyse d'échantillons sanguins, céphalo-rachidiens ou urinaires, qui sont stériles chez un individu sain, ou encore dans le domaine environnemental.

En revanche dans le cas d'autres échantillons, tels que des échantillons alimentaires par exemple, l'échantillon à analyser peut contenir naturellement une flore microbiologique en plus ou moins grande quantité. C'est notamment le cas dans la plupart des produits laitiers, transformés ou non. Pour vérifier la sécurité d'un tel produit il faut alors rechercher spécifiquement la présence d'espèces bactériennes pathogènes au sein de la flore microbiologique totale. Par exemple, on recherchera souvent les *Listeria* dans un fromage, et les *Salmonella* dans de la viande de volailles.

L'aspect quantitatif est également important puisqu'il peut être nécessaire d'évaluer la concentration d'une catégorie de microorganismes d'intérêt dans un échantillon tel qu'un aliment. Ces données varient d'un pathogène à un autre (*cf.* le règlement n° 2073/2005 du journal officiel de l'Union Européenne).

Pour effectuer cette recherche de bactéries pathogènes dans un échantillon, il est le plus souvent nécessaire de disperser et/ou diluer l'échantillon dans un milieu de culture liquide adéquat (généralement au 1/10ème), au sein d'un conteneur. L'échantillon est donc pesé, la quantité de liquide d'enrichissement adéquat est ajoutée, puis l'ensemble est homogénéisé. Cette homogénéisation est nécessaire pour garantir la dispersion de la matrice alimentaire dans le milieu de culture et la répartition homogène des microorganismes recherchés. Cette étape est généralement conduite grâce à une action manuelle ou mécanique à travers des conteneurs souples (des sacs) permettant une homogénéisation. L'ensemble de ces étapes (pesée, dilution, homogénéisation) représente la «préparation de l'échantillon ». Le mélange obtenu est ensuite incubé à température donnée (le plus souvent 37°C), pendant une durée déterminée (le plus souvent une nuit) permettant aux microorganismes recherchés de se multiplier, ce que l'on appelle la « phase d'enrichissement ». A l'issue de cette phase d'enrichissement, un prélèvement est généralement effectué en ouvrant le conteneur, pour réaliser l'analyse ultérieure de l'échantillon en-dehors du conteneur ; cette analyse peut par exemple être la détection d'une bactérie pathogène. Dans le cas des échantillons solides ou semi-solides, il peut être utile d'utiliser un filtre, parfois inclus directement dans le sac, qui permettra de retenir les particules solides qui pourraient gêner l'analyse ou le prélèvement qui permettra l'analyse, tout en laissant passer les microorganismes.

L'obtention d'un résultat nécessite donc une suite d'opérations et de manipulations consommatrices de temps et de main d'œuvre et est source de risques éventuels (par exemple contamination de l'opérateur, de l'environnement de travail ou de l'échantillon) ou source d'erreurs. De plus, il est intéressant pour les industriels de pouvoir disposer le plus tôt possible des résultats de ces analyses. Or, la détection des microorganismes recherchés étant réalisée à la fin de la période d'enrichissement, le résultat ne peut être obtenu qu'à l'issue de cette phase d'enrichissement et des étapes de détection qui la suivent.

Le document FR 2691374 décrit un sac filtrant constitué de deux parois en matériau déformable 1 et 2 et d'un filtre 3 soudés ensemble sur les trois côtés, le quatrième côté étant laissé ouvert de manière à former un sac. Le filtre est soudé entre les deux parois de manière à former deux compartiments, l'un contenant la solution mélange brut et l'autre contenant le filtrat. Ce sac nécessite d'être ouvert pour réaliser un prélèvement puis une analyse. Cette opération est difficilement automatisable et augmente le risque de contamination de l'échantillon et du manipulateur.

Le document FR 2938449 présente un sac à filtre pour la microbiologie disposant d'un canal permettant de faire remonter le filtrat jusqu'au côté ouvert du sac et ainsi le prélever simplement. Dans ce dispositif le sac n'est pas fermé, il doit être ouvert pour permettre au filtrat de remonter le canal. En outre le canal ne permet pas de distribuer facilement le contenu du sac ou d'y connecter de manière étanche un capteur ou un système de prélèvement automatique.

Le document US 5,266,486 présente un conteneur permettant de détecter des microorganismes présents dans un échantillon, par exemple dans un échantillon sanguin, en détectant des métabolites issus de la croissance de ces microorganismes. Bien que ce conteneur permette de détecter indirectement la présence de bactéries, éventuellement sans avoir à l'ouvrir, il ne permet pas une homogénéisation mécanique de l'échantillon à analyser car ce conteneur est forcément solide. Cela limite donc son utilisation aux échantillons liquides.

Le document WO 2004/092401 décrit un procédé de détection de bactéries présentes dans un échantillon ainsi qu'un dispositif de détection capable de détecter de manière spécifique des bactéries présentes dans un échantillon au cours de la phase d'enrichissement sans prélèvement de la part de l'opérateur. Cependant ce procédé présente plusieurs inconvénients : il nécessite l'ouverture du sac après homogénéisation pour y introduire le dispositif de détection, le dispositif n'étant pas adapté aux appareils d'homogénéisation mécanique ; de plus, il nécessite un contrôle des températures qui doivent être différentes dans les deux compartiments du dispositif.

Le document WO 2012/004540 décrit une méthode permettant la détection spécifique de microorganismes dans un échantillon pendant la phase d'enrichissement. Dans cette méthode, un biocapteur est scellé dans un conteneur plastique souple ou plongé dans le milieu de culture. Le capteur étant scellé au niveau de l'échantillon il n'est pas possible d'homogénéiser le contenu du sac sans risquer d'endommager le capteur ou de gêner l'homogénéisation. Dans la seconde alternative proposée dans le document WO 2012/004540, le biocapteur doit être enfermé dans le sac. Cela impose des contraintes de communication avec l'extérieur, notamment parce que le positionnement du biocapteur dans le sac n'est pas reproductible.

Le document WO 2011/133694 décrit une méthode électrochimique ainsi qu'un dispositif spécifique pour la détection de *Salmonella enterica* par la mise en œuvre d'aptamères. Cependant, ce dispositif, mettant en œuvre un conteneur d'échantillon rigide, nécessite également une homogénéisation préalable de l'échantillon, surtout si celui-ci est solide ou semi-solide, et l'intervention d'un opérateur pour effectuer le transfert de la suspension à analyser dans le conteneur. Il ne permet donc pas d'effectuer les étapes successives de préparation et d'enrichissement au sein du même conteneur, sans ouvrir ce dernier à l'issue de l'homogénéisation de l'échantillon.

Le document US 5 266 486 A décrit une méthode, un capteur, et un appareil pour la détection d'activité biologique dans un échantillon.

Le document WO 2012/085470 A1 décrit un récipient à usage biopharmaceutique comprenant un conteneur flexible, ayant une paroi, un espace intérieur pour recevoir un contenu biopharmaceutique liquide ou pâteux.

Le document US 2011/217767 Aldécrit des bioréacteurs stationnaires et des récipients de mélange équipés de sacs flexibles à usage unique utilisable dans tous les types de procédés et produits biologique.

Il n'existe donc pas de procédé permettant de préparer, transporter, diluer, homogénéiser et analyser un échantillon solide ou semi-solide qui puisse être facilement automatisable et diminue les risques de contamination.

La Demanderesse a développé une méthode d'analyse d'un échantillon permettant de pallier les inconvénients des méthodes connues jusque-là. Ainsi, la présente invention concerne une méthode d'analyse d'un échantillon, un conteneur souple, ainsi qu'un kit d'analyse.

Les Fig.la à le représentent différents modes de réalisation d'un conteneur souple.

Les Fig.2a à 2e, 3 et 4 illustrent la mise en œuvre de la méthode selon l'invention.

Les figures 5a à 5c représentent des résultats obtenus pour la recherche simultanée des *Salmonella spp* et *Escherichia coli* O157:H7 dans trois échantillons de viande hachée de bœuf contenus dans des conteneurs A, B, et C, ceux-ci contenant respectivement des *Salmonella spp*., des *Escherichia coli* O157:H7 et aucun contaminant.

La portée de la protection est définie seulement par les revendications annexées. Ci-après, la terminologie « mode de réalisation » concerne seulement la divulgation et ne fait partie de l'invention que si elle entre dans le champ de protection des revendications.

La méthode d'analyse d'un échantillon selon l'invention comprend :
a) l'introduction de l'échantillon et d'un milieu de culture dans un conteneur souple ;
b) l'homogénéisation de l'échantillon et du milieu de culture de façon à obtenir un mélange homogénéisé ;
c) l'enrichissement du mélange homogénéisé ; et
d) l'analyse du mélange homogénéisé par un système d'analyse ;
caractérisée en ce que
les étapes a) à c) sont effectuées dans un conteneur unique ;
le conteneur souple est fermé à l'issue d'une des étapes a) ou b) ;
un module est connecté au conteneur souple au début, au cours ou à la fin de l'étape c) ; et
les étapes c) et d) et la connexion du module au conteneur souple sont réalisées sans nécessiter la réouverture du conteneur,
le conteneur souple comprenant une zone d'homogénéisation et une prise située hors de la zone d'homogénéisation, ladite prise étant étanche lorsqu'elle n'est pas connectée et permet de laisser passer le mélange homogénéisé lorsqu'elle est connectée à une prise du module.
Selon la divulgation l'homogénéisation n'est pas qu'une homogénéisation par simple agitation du conteneur et/ou du contenu. Par homogénéisation, on entend aussi une homogénéisation du contenu par déformation mécanique du conteneur. Une telle homogénéisation par déformation mécanique du conteneur englobe des opérations d'écrasement, de broyage, malaxage, mixage lorsque le contenu est solide, semi-solide ou pâteux mais également des opérations de mélangeage de liquides ou de suspensions.

Selon la divulgation, plusieurs conteneurs sont connectés à un module lors de la méthode d'analyse permettant ainsi l'analyse simultanée des mélanges contenus dans chacun des conteneurs.

Cette méthode a pour avantage de réaliser toutes les étapes dans un conteneur unique y compris l'homogénéisation de l'échantillon sans nécessiter la réouverture du conteneur après l'étape d'enrichissement. Ainsi, le nombre d'interventions d'un opérateur et l'exposition de l'opérateur au mélange homogénéisé une fois enrichi peuvent être réduits, ce qui permet de limiter les risques de contamination et également d'automatiser plus facilement le procédé. Cette méthode peut également être facilement automatisée, du fait que le conteneur ne nécessite pas d'être ré-ouvert une fois que l'étape d'enrichissement a démarré. Il est ainsi possible de manipuler facilement les sacs avec un automate et de réaliser les opérations de collecte et/ou d'analyse, alors qu'elles sont aujourd'hui effectuées essentiellement manuellement à cause de la ré-ouverture nécessaire du conteneur.

Dans l'étape a) de préparation de l'échantillon, un échantillon d'un poids déterminé à analyser est introduit dans un conteneur souple.

La nature et la consistance de l'échantillon à analyser ne sont pas particulièrement limitées. L'échantillon peut être liquide ou au moins en partie pâteux ou solide. L'échantillon peut être par exemple issu de produits agroalimentaires tels que les produits à base de viande, les produits à base de poisson, les produits laitiers, les produits à base d'oeuf ou les produits végétaux. Il peut également s'agir de prélèvements environnementaux (eau de production, surfaces de travail) ou d'échantillons de production primaires (fèces d'animaux, pédichiffonnettes, eaux de couvoirs). L'échantillon peut également provenir de prélèvements humains (sang, urine, fèces, expectorations pulmonaires, etc.) ou encore des échantillons issus de l'industrie cosmétique et pharmaceutique. Un milieu de culture est ajouté à l'échantillon. Le milieu de culture est choisi parmi les milieux de cultures les plus adaptés de façon à favoriser le développement de l'analyte cible. A titre d'exemples non limitatifs, le milieu de culture peut être choisi parmi l'eau peptonnée tamponnée (EPT), le bouillon trypto-caséine soja (TSB), le bouillon d'infusion cœur-cervelle (BHI), le Fraser-demi, le bouillon BOLTON.

Le conteneur souple peut être tout conteneur souple adapté notamment à une homogénéisation mécanique. Au sens de la présente invention, le terme « conteneur souple » ou « sac » est défini notamment comme un conteneur fait de matière souple, tel qu'un plastique souple, et s'ouvrant par le haut. Typiquement, le conteneur de la présente invention comprend deux feuilles de matière plastique polygonales, de préférence essentiellement rectangulaires. Celles-ci sont soudées entre elles par leurs côtés, un côté des feuilles étant libre pour former l'ouverture. Le côté du conteneur présentant l'ouverture est défini comme le haut du conteneur. Le côté opposé à l'ouverture est défini comme le fond du conteneur. Les côtés restants sont définis comme les côtés latéraux du conteneur. Dans la suite de la description, une partie supérieure du conteneur fera référence à une partie du conteneur située du côté de l'ouverture et une partie inférieure du conteneur fera référence à une partie du conteneur située du côté du fond du conteneur. Le fond du conteneur peut former un soufflet. Cependant, afin de permettre une homogénéisation idéale, le fond du conteneur ne présente pas de soufflet et est formé d'une simple soudure de deux feuilles de matière plastique. Le conteneur de la méthode de la présente invention est par exemple un conteneur de type sac d'homogénéisation adapté aux appareils d'homogénéisation de type Stomacher®. Le conteneur présente de préférence un rapport hauteur sur largeur compris entre 1 et 2,5. Par exemple, les dimensions du conteneur sont choisies parmi une hauteur comprise entre 12 et 18 cm pour une largeur comprise entre 7 et 12 cm (15*10 ±2 cm), une hauteur comprise entre 25 et 35 cm pour une largeur comprise entre 14 et 24 cm (30*19 ±5 cm), et une hauteur comprise entre 48 et 68 cm pour une largeur comprise entre 28 et 48 cm (58*38 ±10 cm).

L'ouverture du conteneur doit être suffisamment grande pour permettre l'introduction de l'échantillon à analyser dans le conteneur. Elle doit permettre le remplissage du conteneur souple avec des matières liquides, semi-solides ou solides. Cette ouverture peut être de plus de 2cm de périmètre, voire 5cm, 10 cm, ou plus de 15cm de périmètre, en fonction de la taille de l'échantillon à analyser. Elle peut se situer sur toute ou partie du côté supérieur du conteneur.

Le conteneur souple doit par ailleurs pouvoir être connecté à un module sans nécessiter sa réouverture à l'issue de l'étape d'enrichissement. Par « réouverture du conteneur », on entend une action sur le conteneur qui conduit à une exposition du contenu du conteneur à l'environnement extérieur. Par « module », on entend un système d'analyse pouvant être connecté directement au conteneur souple ou un système de collecte en vue d'une analyse ultérieure.

Ainsi, le conteneur comprend une prise. Par « prise » au sens de la présente invention, on entend un moyen mécanique rigide muni d'un élément de connexion permettant de lier physiquement, de manière étanche et de préférence de manière réversible le conteneur au système d'analyse. La prise présente un état étanche lorsqu'elle n'est pas connectée et un état passant, permettant de laisser passer le mélange homogénéisé, une fois connectée au module. Plus préférentiellement, la prise permet une connexion réversible, c'est-à-dire qu'elle devient à nouveau étanche lorsque le module est déconnecté. Ainsi, il est possible de changer de module pour effectuer par exemple une mesure de confirmation, une autre analyse ou effectuer un prélèvement tout en évitant les risques de contamination. Plus particulièrement, la prise du conteneur est par exemple constituée d'un élément femelle, ou respectivement mâle, destiné à coopérer avec un élément mâle, ou respectivement femelle, d'une prise complémentaire située sur le module. De préférence, la prise comprend un corps solide permettant la préhension du conteneur souple. Ainsi, le conteneur peut être manipulé facilement par des automates. De préférence, la manipulation du sac est réalisée par un automate au cours d'au moins une des étapes a) à d), plus préférentiellement au moins au cours lors des étapes b) à d).

Dans un mode de réalisation particulier, l'ouverture est munie de moyens de connexion et la prise est située sur un bouchon, lui-même muni de moyens de connexion aptes et destinés à être connectés aux moyens de connexion de l'ouverture. Par moyens de connexion, on entend tout connecteur permettant de lier physiquement, de manière étanche et éventuellement de manière réversible deux éléments entre eux. Ils sont généralement formés de deux parties complémentaires disposées chacune sur un des éléments destinés à être liés entre eux, ces deux parties complémentaires étant aptes et destinées à être connectées entre elles. Les moyens de connexion peuvent être des moyens de connexion mécanique, tels que des pas de vis, des systèmes d'attache de type clips.

Le conteneur peut comprendre un ou plusieurs filtres sur toute ou partie de la surface. Notamment, le filtre est formé d'une feuille soudée entre les deux feuilles de matière plastique formant le conteneur. Le filtre peut être également formé d'une poche soudée au conteneur au niveau de l'ouverture. Le filtre sépare l'intérieur du conteneur en deux volumes, de préférence, le premier volume débouche sur l'ouverture et le second débouche sur la prise. Le filtre présente une porosité suffisante pour retenir les particules solides. Le filtre présente de préférence une porosité comprise entre 50 et 500 µm.

L'étape b) d'homogénéisation peut être effectuée mécaniquement par un appareil d'homogénéisation ou manuellement en pressant et en secouant le conteneur. Une homogénéisation mécanique est particulièrement adaptée pour l'analyse d'échantillons essentiellement solides ou pâteux. De préférence, l'homogénéisation est réalisée par un appareil d'homogénéisation mécanique bien connu de l'homme du métier tel qu'un Stomacher®. A l'issue de cette étape d'homogénéisation, l'échantillon et le milieu de culture forment un mélange homogénéisé encore appelé suspension.

Afin de pouvoir réaliser cette homogénéisation, le conteneur comprend une zone d'homogénéisation. Le terme « zone d'homogénéisation » définit une zone du conteneur utile à une homogénéisation, notamment une homogénéisation mécanique à l'aide d'appareils d'homogénéisation de type Stomacher®. Cette zone d'homogénéisation est généralement située en partie inférieure du conteneur, typiquement dans la moitié inférieure, voire le tiers inférieur du conteneur. Par exemple, la zone d'homogénéisation est définie par un rectangle ayant trois de ses côtés correspondant au fond du conteneur et aux côtés latéraux du conteneur, le quatrième côté correspondant à une droite située à 10 à 50 cm, de préférence 15 à 40 cm au-dessus du fond du conteneur. La surface de la zone d'homogénéisation correspond généralement à 30 à 90%, de préférence 50 à 80%, de la surface du conteneur souple. Par exemple, pour un conteneur de dimension 15*10 cm ±2 cm, la zone d'homogénéisation a une dimension de 10*10 ±2 cm ;pour un conteneur de dimension 30*19cm ±5 cm, la zone d'homogénéisation a une dimension de 19*19 ±5 cm; et pour un conteneur de dimension 58*38 ±10 cm, la zone d'homogénéisation a une dimension de 38*38 ±10 cm. Par ailleurs, la zone d'homogénéisation définit de préférence un volume supérieur à 90 cm³ lorsque le conteneur est rempli. Par exemple, pour un conteneur de dimension 15*10cm ±2 cm, la zone d'homogénéisation définit de préférence un volume compris entre 90 et 120 cm³ ; pour un conteneur de dimension 30*19cm ±5 cm, la zone d'homogénéisation définit de préférence un volume compris entre 220 et 280 cm³ ; et pour un conteneur de dimension 58*38 ±10 cm, la zone d'homogénéisation définit de préférence un volume compris entre 3370 et 3490 cm³.

La prise du conteneur souple doit être située en dehors de la zone d'homogénéisation. Elle peut être située sur la partie supérieure ou sur la partie inférieure du conteneur. De préférence, la prise est située en partie supérieure du conteneur au-dessus de la zone d'homogénéisation, par exemple au voisinage de l'ouverture. La prise peut être située sur un appendice s'étendant à partir de l'un des côtés du conteneur. Bien que situé de préférence sur un des côtés latéraux du conteneur, l'appendice peut également être situé sur le fond du conteneur. La prise peut être isolée de l'intérieur du conteneur par des moyens de séparation. Les moyens de séparation peuvent être constitués par un dispositif distinct du conteneur, tels qu'une pince ou une baguette, ou être intégrés au conteneur, tels qu'une fermeture « zip » ou une fermeture à glissière.

La fermeture du conteneur souple à l'issue des étapes a) ou b) peut être effectuée par tout moyen de fermeture permettant une fermeture étanche ou quasi-étanche du conteneur. Par fermeture quasi-étanche, on entend une fermeture étanche notamment aux agents pathogènes mais pouvant être sensiblement perméable aux gaz. Les moyens de fermeture peuvent être constitués par un dispositif de fermeture distinct du conteneur, tels qu'une pince ou une baguette du type BagClip® commercialisé par Interscience. Ils peuvent être également intégrés au conteneur. Dans ce cas, les moyens de fermeture peuvent être une fermeture « zip » ou une fermeture à glissière formée par exemple par un ensemble de nervures et de rainures disposées sur les surfaces internes du sac au niveau de l'ouverture, et destinées à coopérer entre elles pour assurer une fermeture étanche, comme par exemple sur les sac Minigrip commercialisés par Flexico. Les moyens de fermeture peuvent également être constitués par un bouchon correspondant à l'ouverture. Dans ce cas, le bouchon et l'ouverture sont de préférence pourvus de moyens de connexion aptes et destinés à être connectés entre eux. La fermeture peut être également réalisée en soudant thermiquement les parois du conteneur au niveau de l'ouverture.

L'étape c) d'enrichissement consiste à placer le conteneur souple contenant le mélange homogénéisé dans des conditions favorisant le développement de l'analyte cible. Typiquement, le conteneur souple est placé dans une étuve entre 20 et 45 °C, pour des analyses classiques en microbiologie.

Un module est connecté au conteneur souple au début, au cours ou à la fin de l'étape c), c'est-à-dire avant, pendant ou après l'enrichissement. Pour cela, le module selon l'invention comprend une prise apte et destinée à être connectée avec la prise du conteneur souple. Une fois les prises du conteneur souple et du module connectées entre elles, le module peut accéder au contenu du conteneur souple. En d'autres termes, le conteneur souple et le module sont en communication de façon à ce que le mélange homogénéisé puisse passer librement de l'intérieur du conteneur au module, sans ouvrir le conteneur. De préférence, la prise est positionnée vers le bas lorsque le système d'analyse est connecté au conteneur. Ainsi, un changement de configuration du conteneur souple peut être nécessaire afin que le mélange homogénéisé soit en contact avec la prise. Notamment, lorsque la prise est située en partie supérieure du conteneur, il peut être nécessaire de positionner le conteneur sensiblement horizontalement sur un des côtés latéraux, voire de retourner complètement le conteneur de façon à ce que le mélange homogénéisé contenu dans le conteneur vienne en contact avec la prise. Dans certains cas, le module peut également être connecté au conteneur souple avant l'étape a) ou l'étape b) pour autant que le module ne risque pas d'être endommagé lors de l'homogénéisation.

A l'étape d), l'analyse du mélange homogénéisé est réalisée par un système d'analyse. De préférence, l'analyse est réalisée en continu, c'est-à-dire en temps réel ou à intervalles de temps réguliers, par exemple au cours de l'enrichissement. Dans ce cas, l'étape d) peut être réalisée au cours de l'étape c).

Selon l'invention le module comprend le système d'analyse ou une partie de celui-ci. Le système d'analyse peut donc être connecté directement au conteneur par la prise du module. Le système d'analyse peut être un système d'analyse ponctuel. Dans ce cas, le module est connecté de préférence avant ou après l'étape d'enrichissement. Le système d'analyse peut également être un système d'analyse en continu. Dans ce cas, le module est connecté au conteneur de préférence avant l'étape d'enrichissement et reste connecté au cours de l'étape d'enrichissement.

Dans une seconde divulgation, le module comprend un système de collecte. Ainsi, un prélèvement du mélange homogénéisé est réalisé par le système de collecte lors de la connexion du module au conteneur souple, en vue d'une analyse ultérieure par le système d'analyse. Dans ce cas, le système de collecte est connecté de préférence avant ou après l'étape d'enrichissement, toujours sans ouverture du sac.

Le système d'analyse peut être tout système permettant l'analyse d'un échantillon, notamment une analyse biologique, chimique, physico-chimique ou microbiologique. De préférence, le système d'analyse est un système d'analyse microbiologique, notamment un ensemenceur de milieux gélosés, un automate de biologie moléculaire, un automate d'analyse immuno-enzymatique, un spectromètre de masse, un cytomètre en flux, un test immuno-chromatographique, un capteur électrochimique, piézo-électrique ou optique. Le système d'analyse met de préférence en œuvre une méthode optique permettant la détection directe d'un analyte en temps réel sans marqueur, telle que la résonance des plasmons de surface, les méthodes basées sur l'interférométrie ou les guides d'onde optiques, ou la microbalance à quartz. A cet effet, le système d'analyse comprend avantageusement des moyens de détection. Le système d'analyse peut également comprendre un filtre disposé en amont des moyens de détection pour protéger les moyens de détection d'éventuels dépôts de particules solides. Le système d'analyse peut également comprendre une unité de mesure permettant la mesure et éventuellement l'interprétation des signaux détectés par les moyens de détection. Dans ce cas, l'unité de mesure peut être distincte du module selon l'invention comprenant les moyens de détection.

Dans un mode de réalisation particulier, les moyens de détection comprennent un ou plusieurs biocapteurs spécifiques permettant la détection sélective d'un analyte cible spécifique, notamment des biocapteurs optiques ou électrochimiques. Dans la présente demande, un biocapteur est constitué d'au moins un ligand susceptible de détecter et/ou d'interagir avec un analyte, des moyens de transductions qui transforme le signal chimique ou biologique en un signal physique facilement interprétable. Les moyens de transduction peuvent être basés, de manière non exhaustive, sur un procédé électrochimique, optique, acoustique, calorimétrique, électromagnétique ou encore piézoélectrique. Les biocapteurs peuvent être fonctionnalisés par des ligands spécifiques notamment choisis parmi les anticorps, les aptamères, les protéines de phage, les antigènes, les oligosaccharides et les petites molécules organiques. Par exemple, un biocapteur tel que décrit dans les documents WO 2012/073202 ou WO 2013/060989 peut être utilisé comme moyens de détection selon l'invention. Les biocapteurs peuvent comprendre plusieurs zones de détection pouvant comprendre des capteurs différents. Les différentes zones de détection peuvent être dirigées contre le même analyte cible ou contre des analytes cibles différents. Une ou plusieurs zones de détection peuvent également servir de référence (témoin négatif).

L'analyte cible peut être choisi parmi les bactéries ainsi que les protéines, les polysaccharides, les acides gras et les acides nucléiques qui les composent ou qu'elles émettent. De préférence, l'analyte cible est choisi parmi les bactéries pathogènes telles que les *Salmonella*, *Listeria* et les souches entéro-hémorragiques d'*Escherichia coli* et leurs métabolites protéiques, notamment les toxines protéiques.

Dans la divulgation, les moyens de détection sont situés directement sur la prise du module de façon à ce que, lorsque le module est connecté au conteneur, les moyens de détection soient à l'intérieur ou en communication directe avec l'intérieur du conteneur. Ainsi, l'étape d'analyse peut être réalisée directement dans le conteneur. De préférence, la prise du module comprend une tige sur laquelle sont situés les moyens de détection, et qui pénètre à l'intérieur du conteneur une fois le module connecté au conteneur. Lors de l'étape d'analyse, le conteneur est positionné de façon à ce que les moyens de détection se trouvent au milieu du mélange homogénéisé. Ainsi, le contact des moyens de détection avec les particules solides qui sédimentent ou surnagent peut être évité.

Dans un mode de réalisation particulier, le conteneur peut comprendre plusieurs prises pour permettre par exemple une connexion de plusieurs systèmes d'analyse et réaliser plusieurs analyses simultanément sur un même mélange homogénéisé.

Dans la divulgation, le système d'analyse peut être connecté simultanément à plusieurs conteneurs pour permettre par exemple une analyse de plusieurs mélanges homogénéisé.

De plus, lorsque l'unité de mesure est distincte du module comprenant les moyens de détection, l'unité de mesure peut être partagée par plusieurs modules. Il est ainsi possible de réaliser l'analyse de plusieurs échantillons en continu avec une unité de mesure, celle-ci réalisant de manière séquentielle des mesures à intervalle de temps régulier auprès de chacun des modules.

Selon un autre mode de réalisation, plusieurs conteneurs sont connectés à un module unique permettant une analyse simultanée des mélanges contenus dans chacun des conteneurs.

La méthode décrite est particulièrement adaptée à l'analyse d'un échantillon au moins en partie solide ou pâteux nécessitant une homogénéisation mécanique. Dans un mode de réalisation préféré, la méthode d'analyse d'un échantillon comprend :
a) l'introduction de l'échantillon et d'un milieu de culture dans un conteneur souple ;
b) l'homogénéisation manuelle ou mécanique de l'échantillon par un appareil d'homogénéisation de façon à obtenir un mélange homogénéisé de l'échantillon dans le milieu de culture ;
c) l'enrichissement du mélange homogénéisé ; et
d) l'analyse du mélange homogénéisé par un système d'analyse.
caractérisée en ce que
les étapes a) à d) sont effectuées dans un conteneur unique ;
le conteneur souple est fermé à l'issue d'une des étapes a) ou b) ;
le système d'analyse est connecté directement au conteneur souple avant l'étape c), et les étapes c) et d) et la connexion du système d'analyse au conteneur souple sont réalisées sans nécessiter la réouverture du conteneur.

Plus préférentiellement, la présente divulgation concerne une méthode d'analyse microbiologique d'un échantillon par détection d'un analyte comprenant :
a) l'introduction de l'échantillon et d'un milieu de culture dans un conteneur souple ;
b) l'homogénéisation manuelle ou mécanique de l'échantillon et du milieu de culture par un appareil d'homogénéisation de façon à obtenir un mélange homogénéisé ;
c) l'enrichissement du mélange homogénéisé ; et
d) l'analyse du mélange homogénéisé par un système d'analyse comprenant des moyens de détection ;
caractérisée en ce que
les étapes a) à d) sont effectuées dans un conteneur unique ;
le conteneur souple est fermé à l'issue d'une des étapes a) ou b) ;
le système d'analyse est connecté directement au conteneur souple au début de l'étape c) ;
le mélange homogénéisé est en contact avec les moyens de détection au cours de l'étape c) et l'analyse du mélange homogénéisé est réalisée en continu au cours de l'étape c) ; et les étapes c) et d) et la connexion du système d'analyse au conteneur souple sont réalisées sans nécessiter la réouverture du conteneur.

La présente divulgation porte également sur un conteneur souple tel que décrit ci-dessus. Le conteneur souple comprend une zone d'homogénéisation, une ouverture et une prise, ladite prise étant située hors de la zone d'homogénéisation. Typiquement, le conteneur souple a une forme essentiellement rectangulaire définie par un côté supérieur comprenant l'ouverture, deux côtés latéraux adjacent au côté supérieur, et un fond opposé au côté supérieur. La zone d'homogénéisation est généralement située en partie inférieure du conteneur. Elle correspond par exemple à un rectangle représentant 30 à 90%, de préférence 50 à 80% de la surface du conteneur souple, un des côté de la zone d'homogénéisation étant de préférence confondu avec le fond du conteneur. La prise est située hors de la zone d'homogénéisation. Elle est située généralement sur un des côtés supérieurs, latéraux ou sur le fond du conteneur. De préférence, la prise est située sur un des côté latéraux du conteneur, à proximité du côté supérieur. Dans un mode de réalisation particulier, l'ouverture du conteneur est munie de moyens de connexion et la prise est située sur un bouchon muni de moyens de connexion aptes et destinés à être connectés aux moyens de connexion de l'ouverture.

Dans un mode de réalisation particulier, la prise comprend un corps solide permettant la préhension du conteneur souple. Ainsi, le conteneur peut être manipulé facilement par des automates. Par conséquent, il est également décrit l'utilisation d'au moins un conteneur souple dans une méthode d'analyse, caractérisée en ce que la manipulation dudit conteneur souple est réalisée par un automate.

Les figures la à le illustrent différents modes de réalisation d'un conteneur. Le conteneur souple **100**, formé de deux feuilles en plastique souple de forme essentiellement rectangulaires soudées entre elles, présente une ouverture **101** sur le côté supérieur du sac, un fond **102** opposé à l'ouverture **101** et deux côtés latéraux **103** et **104**. Le conteneur souple comprend une zone d'homogénéisation **105** destinée à permettre l'homogénéisation de son contenu, notamment par un appareil d'homogénéisation mécanique.

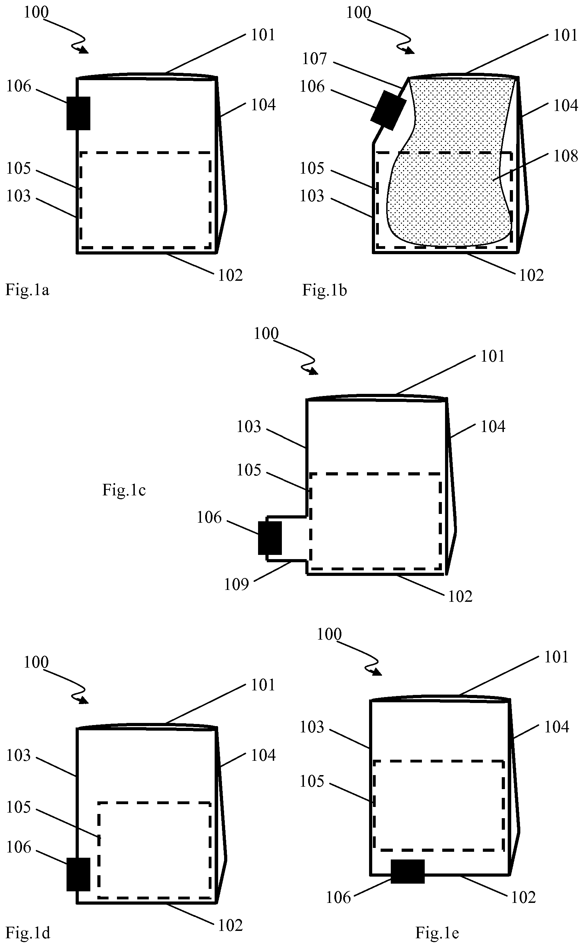

Dans un premier mode de réalisation, la zone d'homogénéisation **105** est située en partie inférieure du conteneur **100**. Elle couvre une zone essentiellement rectangulaire s'étendant dans une première direction d'un côté latéral **103** à l'autre côté latéral **104** du conteneur **100**, et dans la seconde direction depuis le fond du conteneur **104** jusqu'à une limite supérieure située par exemple à une distance comprise entre 10 et 50 cm du fond du conteneur. Dans une première alternative illustrée par la Fig.la, le conteneur **100** comprend une prise **106** située en partie supérieure du côté latéral **103**, au-dessus de la zone d'homogénéisation **105**. Dans une deuxième alternative illustrée par la Fig.1b, le conteneur **100** comprend un coin tronqué **107** entre le côté de l'ouverture **101** et un des côtés latéraux **103**, le coin tronqué **107** étant au-dessus de la zone d'homogénéisation **105**. Une prise **106** est située sur le coin tronqué **107**. Le conteneur **100** comprend en outre un filtre **108**, soudé par exemple au niveau de l'ouverture **101**, le filtre séparant l'intérieur en deux volumes, le premier débouchant sur l'ouverture **101** et le second débouchant sur la prise **106**. Dans une troisième alternative illustrée par la Fig.1c, le conteneur **100** comprend un appendice **109** sur un des côtés latéraux **103**. L'appendice peut être situé en partie inférieure du conteneur à proximité du fond du conteneur **102**. Une prise **106** est située sur l'appendice **109**. L'appendice peut être isolé de l'intérieur du conteneur par des moyens de séparation (non représentés). Le conteneur peut comprendre un filtre (non représenté). Le filtre peut être soudé par exemple au niveau de l'ouverture **101**, comme illustré à la Fig.lb, ou peut être situé à l'entrée de l'appendice **109**.

Dans un deuxième mode de réalisation illustré par la Fig.1d, la zone d'homogénéisation **105** est située en partie inférieure du conteneur **100**. Elle couvre une zone essentiellement telle que décrite dans le premier mode de réalisation mais exclue un des côtés latéraux **103**. Une prise **106** est située sur le côté latéral **103** hors de la zone d'homogénéisation **105**. Le conteneur peut comprendre un filtre (non représenté) comme illustré à la Fig.1b.

Dans un troisième mode de réalisation illustré par la Fig.le, la zone d'homogénéisation **105** est située au-dessus du fond du conteneur **102**. Elle couvre une zone essentiellement rectangulaire s'étendant dans une première direction d'un côté latéral **103** à l'autre côté latéral **104** du conteneur **100**, et dans la seconde direction sur une hauteur comprise par exemple entre 10 et 50 cm. Une prise **106** est située sur le fond du sac **102**, hors de la zone d'homogénéisation. Le conteneur peut comprendre un filtre (non représenté) comme illustré à la Fig.1b.

Le conteneur a l'avantage de ne pas modifier les modes opératoires mis en œuvre dans les laboratoires d'analyses, notamment du point de vue de la préparation de l'échantillon, tout en s'affranchissant de la réouverture du conteneur à l'issue de l'enrichissement, prévenant ainsi toute contamination de l'environnement de travail et des opérateurs.

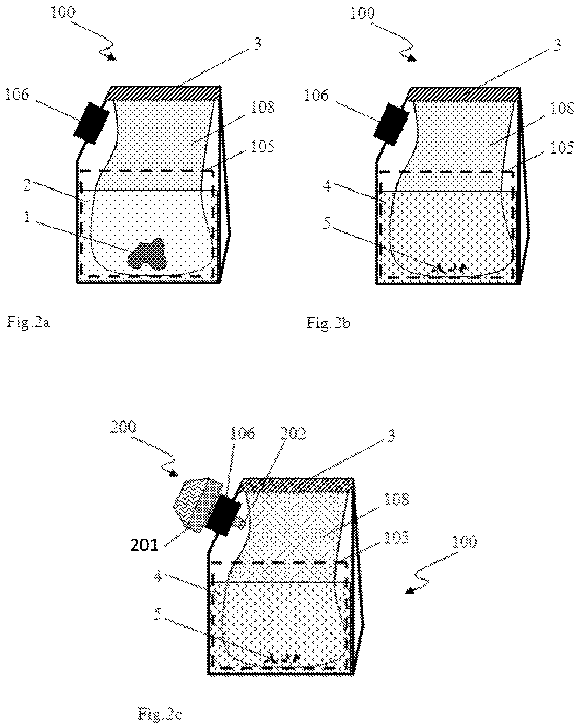

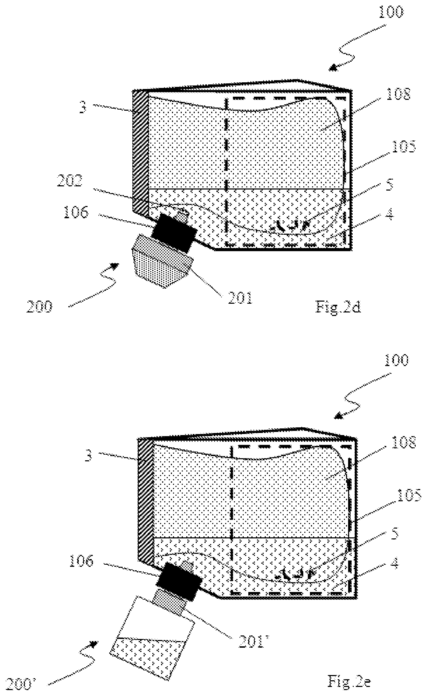

Le conteneur souple est adapté à la méthode d'analyse selon l'invention. Les Fig.2a à 2e illustrent un exemple de mise en œuvre de la méthode selon l'invention. Après l'étape a), le conteneur **100** contient un échantillon solide ou semi-solide **1** et le milieu de culture **2**, et est fermé par une soudure **3**. Il est en position verticale de façon à ce que l'échantillon **1** et le milieu de culture **2** soient dans la zone d'homogénéisation **105**, en partie inférieure du conteneur **100** (Fig.2a). Après l'étape b) d'homogénéisation, l'échantillon **1** et le milieu de culture **2** forment un mélange homogénéisé **4**. Les particules résiduelles **5** issues du broyage de l'échantillon **1** sont retenues par le filtre **108** (Fig.2b). Il est à noter que la soudure **3** aurait très bien pu être réalisée après l'étape b) et non après l'étape a). La prise **201** d'un module **200** comprenant un système d'analyse avec des moyens de détection **202** est connectée à la prise **106** du conteneur **100** au début de l'étape c) (Fig.2c). Dans la Fig. 2c, les moyens de détection sont situés à l'intérieur du conteneur lorsque le système d'analyse est connecté au conteneur. Alternativement, les moyens de détection peuvent être situés dans un compartiment du système d'analyse qui reste à l'extérieur du conteneur mais est en communication directe avec l'intérieur du conteneur lorsque le système d'analyse est connecté au conteneur. Pour permettre l'analyse de la suspension **4** en continu pendant l'étape d'enrichissement c), le conteneur **100** est positionné de façon à permettre au mélange homogénéisé d'être en contact avec les moyens de détection **202**. Par exemple sur la Fig.2d, le conteneur est positionné horizontalement sur le côté latéral **103** adjacent à la prise **106**. Alternativement, le conteneur peut être retourné à 180°, la soudure **3** se retrouvant vers le bas. Lorsque l'analyse est terminée, le module **200** comprenant le système d'analyse peut être remplacé par un module **200'** comprenant un système de collecte (Fig.2e). Ainsi, une fraction du mélange homogénéisé enrichi peut être prélevée pour confirmer le résultat par une autre méthode d'analyse de confirmation, ceci sans que les opérateurs manipulant les conteneurs aient pu être en contact avec le mélange enrichi potentiellement contaminé par des microorganismes pathogènes.

De façon plus spécifique, un conteneur selon la Fig.la peut être utilisé pour l'analyse de 25 mL de lait cru. Le conteneur a par exemple une forme générale rectangle de largeur 17 cm et de hauteur 32 cm (Fig.la). La zone d'homogénéisation **105** mesure 17*18 cm. La prise **106** est située à plus de 18 cm du fond **102**.

Le protocole d'une telle analyse comprend :
- l'introduction de 25 mL de lait cru et de 225 mL d'eau peptonée tamponnée dans le conteneur ;
- le malaxage manuel ou l'homogénéisation mécanique de la zone d'homogénéisation **105** afin d'obtenir un mélange homogénéisé ;
- la fermeture du conteneur à l'aide d'une baguette ou par soudure ;
- la rotation du conteneur de 90° de façon à positionner le côté latéral **103** vers le bas afin de mettre en contact le mélange homogénéisé avec la prise ;
- la connexion d'un module à la prise du conteneur, ledit module comprenant un biocapteur pour la détection en temps réel des *Salmonella* et *Escherichia coli* entéro-hémorragiques. Le biocapteur peut être par exemple un biocapteur, tel que celui décrit dans le document WO 2013/060989, fonctionnalisé avec des ligands *anti-Salmonella, anti-Escherichia coli* entéro-hémorragiques et anti-IgG de lapin (ces derniers étant utilisés comme contrôle négatif) ;
- l'enrichissement du mélange homogénéisé en plaçant le conteneur dans une enceinte thermostatée à 41,5°C ; et
- l'analyse en temps réel des *Salmonella* et *Escherichia coli* entéro-hémorragiques par lecture optique se basant sur le phénomène physique des plasmons de surface comme décrit dans le document EP 2646565.

De même, un conteneur selon la Fig.1b peut être utilisé pour l'analyse de 375 g de viande hachée. Un tel conteneur a par exemple une forme générale rectangulaire de largeur 38 cm et de hauteur 55 cm, tronqué sur un de ses coins supérieurs. Le coin tronqué **107** mesure 18 cm, le côté supérieur comprenant l'ouverture **101** mesure 29 cm et le côté latéral **103** adjacent au coin tronqué mesure40 cm. La prise **106** est située sur le coin tronqué **107** à 8 cm de l'ouverture **101** et la zone d'homogénéisation **105** mesure 38*38 cm. Un filtre présentant une porosité de 250 µm sépare le conteneur en deux volumes, le premier débouchant sur l'ouverture et le second débouchant sur la prise. Le protocole d'une telle analyse comprend :
- l'introduction de 375 g de viande hachée et de 3375 mL d'eau peptonée tamponnée dans le conteneur ;
- le malaxage de la zone d'homogénéisation par un malaxeur à pales de type Stomacher® pendant 60 secondes afin d'obtenir un mélange homogénéisé ;
- la fermeture du conteneur de manière étanche par soudure de l'ouverture de façon à ce que la prise soit située entre 2 et 10 cm de la soudure ;
- la connexion d'un module à la prise du conteneur, ledit module comprenant un biocapteur pour la détection en temps réel des *Salmonella* et *Escherichia coli* entéro-hémorragiques ;
- la rotation du conteneur de 180° de façon à positionner le côté supérieur comprenant l'ouverture **101** vers le bas afin de mettre en contact le mélange homogénéisé avec la prise **106** située sur le coin tronqué **107** pour collecter une fraction du mélange homogénéisé enrichi ;
- l'enrichissement du mélange homogénéisé en plaçant le conteneur dans une enceinte thermostatée à 41,5°C ;
- l'analyse en temps réel des *Salmonella* et *Escherichia coli* entéro-hémorragiques ;
- dans le cas d'un résultat positif, la déconnexion du module comprenant le système d'analyse et la connexion d'un module comprenant un système de collecte pour collecter une fraction du mélange homogénéisé enrichi ;
- l'analyse de la fraction de mélange homogénéisé enrichi par PCR ou toute autre méthode rapide de détection de pathogènes pour confirmer la détection des *Salmonella* et/ou *Escherichia coli* entéro-hémorragiques.

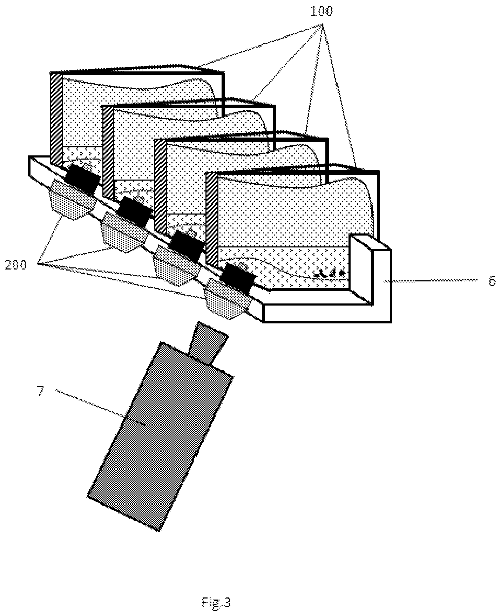

Par ailleurs, la Fig.3 illustre un système d'analyse permettant le suivi de l'analyse à intervalles réguliers de plusieurs conteneurs simultanément. Sur la Fig.3, des modules **200** comprenant des moyens de détection (non représentés) sont connectés à chacun des conteneurs souples **100**. Les conteneurs souples **100** sont disposés sur un support mobile **6** qui présente séquentiellement chacun des modules **200** à une unité de mesure **7** pendant une durée suffisante pour permettre à l'unité de mesure **7** de réaliser une mesure.

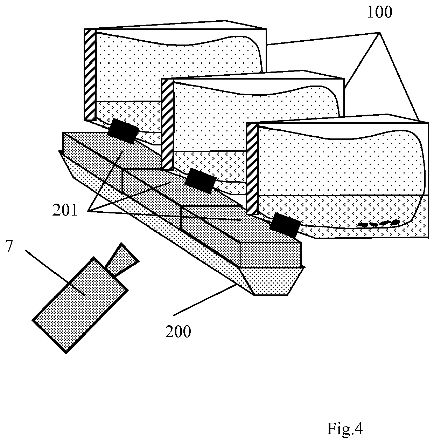

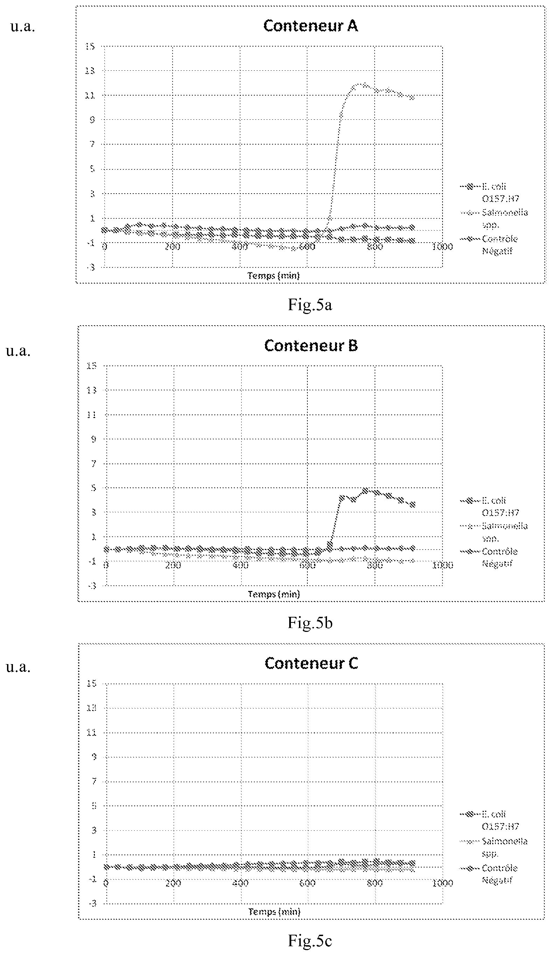

Selon un autre mode réalisation, la Fig.4 illustre un système d'analyse permettant l'analyse simultanée de plusieurs conteneurs **100** connectés à un même module **200** comprenant plusieurs prises **201** et des moyens de détection **202** (non représentés) répartis en autant de zones distinctes et sans communication physique. Ainsi, le mélange de chaque conteneur **100** est connecté à une seule zone comprenant des moyens de détection **202** (non représentés) et l'analyse est réalisée de manière indépendante pour chacun des conteneurs. Une unité de mesure **7** permet alors d'analyser de manière indépendante mais simultanée le mélange homogénéisé de chacun des conteneurs. Ainsi, plusieurs conteneurs connectés à un même module commun selon la Fig.4, peuvent être utilisés pour l'analyse de 25g de viande hachée de bœuf. Les conteneurs ont par exemple une forme générale rectangulaire de largeur 19 cm et de hauteur 35 cm. La prise **106** est située sur le côté **103** à plus de 26 cm du fond **102**. Le module **200** comprenant le système d'analyse porte par exemple trois prises **201** aptes et destinées à être connectées avec les prises **106** des trois conteneurs. Chacune des trois prises communique de manière indépendante avec des moyens de détection **202** (dans ce cas des anticorps dirigés contre *Salmonella spp*, *Escherichia coli* O157:H7 et contre des IgG de lapin).
Le protocole d'une telle analyse comprend :
- l'introduction dans trois conteneurs (A, B, C) de 25g d'échantillon de viande hachée de bœuf et de 225 mL d'eau peptonée tamponnée ;
- L'inoculation du conteneur A avec 10 u.f.c. (unités formant colonies) de *Salmonella* Typhimurium et l'inoculation du conteneur B avec 3 u.f.c. (unités formant colonies) d'*Escherichia coli* O157:H7 (le conteneur C ne reçoit pas de culture microbienne) ;
- le malaxage des zones d'homogénéisation de chacun des conteneurs par un malaxeur à pales de type Stomacher® pendant 60 secondes pour obtenir des mélanges homogénéisés ;
- la fermeture des conteneurs par soudure ;
- la rotation de 180° de chacun des conteneurs de façon à positionner les fonds **102** vers le haut afin de mettre en contact les mélanges homogénéisés avec les prises **201** ;
- la connexion des prises des conteneurs à un module **200** commun, ledit module comprenant des moyens de détection **202** (biocapteurs) pour la détection en temps réel des *Salmonella spp.* et *Escherichia coli* O157:H7 ;
- l'enrichissement des mélanges homogénéisés en plaçant les conteneurs connectés dans une enceinte thermostatée à 37°C ; et
- l'analyse en temps réel des *Salmonella spp.* et des *Escherichia coli* O157:H7 par une méthode d'imagerie par résonance des plasmons de surface comme décrit dans le document EP 2646565.
Les résultats obtenus pour la recherche des *Salmonella spp* et *Escherichia coli* O157:H7 pour les 3 échantillons de viande hachée de bœuf des conteneurs A, B, et C sont représentés en Fig.5a, Fig.5b et Fig.5c respectivement. On y observe que les moyens de détection ont permis de réaliser des détections de différents microorganismes en parallèle, dans chacun des conteneurs simultanément.

La présente invention porte également sur un kit d'analyse comprenant le conteneur souple et un module tels que décrits ci-dessus.

Le kit d'analyse selon l'invention comprend :
- au moins un conteneur souple comprenant une zone d'homogénéisation, une ouverture et une prise, ladite prise étant située hors de la zone d'homogénéisation;
- un module comprenant un système d'analyse ou une partie de celui-ci, une prise apte et destinée à être connectée avec la prise du conteneur souple, ladite prise présentant un état étanche lorsqu'elle n'est pas connectée et un état passant, permettant de laisser passer le mélange homogénéisé, une fois connectée à la prise du module.

Le module est de préférence un module de détection comprenant des moyens de détection tels que défini ci-dessus. Les moyens de détection permettent par exemple la détection sélective d'au moins un analyte cible choisi parmi les bactéries et les protéines. Alternativement, le module peut être un module de collecte comprenant un système de collecte tel que défini ci-dessus.

Le kit selon la divulgation peut être utilisé pour l'analyse microbiologique d'un échantillon, notamment dans le domaine agroalimentaire, l'environnement ou la santé. Le kit selon l'invention est particulièrement adapté à l'analyse d'un échantillon solide ou semi-solide nécessitant une étape d'homogénéisation mécanique, notamment par la mise en œuvre de la méthode selon l'invention.

La présente invention permet d'effectuer toutes les étapes de préparation (notamment l'homogénéisation), d'enrichissement et éventuellement d'analyse dans un conteneur souple unique et sans ouverture du conteneur.

Selon la divulgation, elle permet également d'analyser de manière indépendante et simultanée le mélange homogénéisé dans plusieurs conteneurs grâce à l'utilisation d'un module unique.

Ainsi, non seulement les risques de contamination sont réduits, mais la mise en œuvre de l'analyse est simplifiée du fait de la réduction du nombre d'opérations nécessaires. Cette simplification permet une automatisation plus facile de l'analyse. Dans un mode de réalisation particulier, le kit selon l'invention permet également de réaliser l'analyse en temps réel au cours de l'étape d'enrichissement. Ceci diminue le temps nécessaire pour une réponse positive, qui peut être obtenue dès que la présence de l'analyte cible est détectée. Enfin, le système d'analyse étant fourni séparément du conteneur souple, il est possible de choisir le système d'analyse adapté à l'analyte cible recherché ou éventuellement de changer de système d'analyse par exemple pour confirmer un résultat positif sans réouverture du conteneur.

## Revendications

1. Méthode d'analyse d'un échantillon comprenant :
a) l'introduction de l'échantillon et d'un milieu de culture dans un conteneur souple (100) ;
b) l'homogénéisation de l'échantillon et du milieu de culture de façon à obtenir un mélange homogénéisé ;
c) l'enrichissement du mélange homogénéisé ; et
d) l'analyse du mélange homogénéisé par un système d'analyse,
**caractérisée en ce que**
les étapes a) à c) sont effectuées dans un conteneur unique (100) ;
le conteneur souple (100) est fermé à l'issue d'une des étapes a) ou b) ;
un module (200) comprenant le système d'analyse est connecté au conteneur souple (100) au début, au cours ou à la fin de l'étape c) ;
les étapes c) et d) et la connexion du module (200) au conteneur souple (100) sont réalisées sans nécessiter la réouverture du conteneur;
le conteneur souple (100) comprenant une zone d'homogénéisation (105) et une prise (106) située hors de la zone d'homogénéisation (105), ladite prise (106) étant étanche lorsqu'elle n'est pas connectée et permet de laisser passer le mélange homogénéisé lorsqu'elle est connectée à une prise (201) du module (200).

2. Méthode selon la revendication 1, **caractérisée en ce que** l'analyse est une analyse microbiologique.

3. Méthode selon l'une des revendications 1 ou 2, **caractérisée en ce que**
le conteneur souple (100) comprend une ouverture (101) ;
le module (200) comprend une prise (201) apte et destiné à être connectée avec la prise du conteneur souple (106).

4. Méthode selon la revendication 3, **caractérisée en ce qu'**un changement de conformation du conteneur souple (100) est réalisé après sa fermeture et avant l'étape d) afin que le mélange homogénéisé soit en contact avec la prise (106) destinée à être connectée avec la prise (201) du module (200).

5. Méthode selon l'une des revendications 1 à 3, **caractérisée en ce que** le système d'analyse comprend des moyens de détection (202) permettant la détection sélective d'au moins un analyte cible choisi parmi les bactéries et les protéines.

6. Méthode selon la revendication 5, **caractérisée en ce que** les moyens de détection (202) sont des biocapteurs optiques ou électrochimiques spécifiques fonctionnalisés par des ligands spécifiques, de préférence choisis parmi les anticorps, les aptamères et les protéines de phage, des oligosaccharides ou des petites molécules organiques.

7. Méthode selon l'une des revendications 1 à 6, **caractérisée en ce que** l'échantillon est solide ou semi-solide.

8. Kit d'analyse comprenant :
- au moins un conteneur souple (100) comprenant une zone d'homogénéisation (105), une ouverture (101) et une prise (106), ladite prise étant située hors de la zone d'homogénéisation (105) ;
- un module (200) comprenant un système d'analyse ou une partie de celui-ci, une prise (201) apte et destinée à être connectée avec la prise du conteneur souple (106), ladite prise (106) présentant un état étanche lorsqu'elle n'est pas connectée et un état passant, permettant de laisser passer le mélange homogénéisé, une fois connectée à la prise (201) du module (200).

9. Kit selon la revendication 8, **caractérisé en ce que** le système d'analyse comprend des moyens de détection (202) permettant la détection sélective d'au moins un analyte cible choisi parmi les bactéries ainsi que les protéines, les polysaccharides, les acides gras et les acides nucléiques qui les composent ou qu'elles émettent.

10. Kit selon la revendication 9, **caractérisé en ce que** les moyens de détection (202) sont des biocapteurs optiques ou électrochimiques spécifiques fonctionnalisés par des ligands spécifiques, de préférence choisis parmi les anticorps, les aptamères et les protéines de phage, des oligosaccharides ou des petites molécules organiques.

## Patentansprüche

1. Verfahren zum Analysieren einer Probe, umfassend:
a) Einbringen der Probe und eines Kulturmediums in einen flexiblen Behälter (100);
b) Homogenisieren der Probe und des Kulturmediums, um eine homogenisierte Mischung zu erhalten;
c) Anreichern der homogenisierten Mischung; und
d) Analyse der homogenisierten Mischung durch ein Analysesystem,
**dadurch gekennzeichnet, dass**
die Schritte a) bis c) in einem einzigen Behälter (100) durchgeführt werden;
der flexible Behälter (100) nach einem der Schritte a) oder b) geschlossen wird;
ein Modul (200), das das Analysesystem umfasst, zu Beginn, während oder am Ende von Schritt c) mit dem flexiblen Behälter (100) verbunden wird;
die Schritte c) und d) und der Anschluss des Moduls (200) an den flexiblen Behälter (100) durchgeführt werden, ohne dass der Behälter erneut geöffnet werden muss;
wobei der flexible Behälter (100) einen Homogenisierungsbereich (105) und ein Verbindungsstück (106) aufweist, das außerhalb des Homogenisierungsbereichs (105) angeordnet ist, wobei das Verbindungsstück (106) abgedichtet ist, wenn es nicht angeschlossen ist, und die homogenisierte Mischung durchlässt, wenn es mit einem Verbindungsstück (201) des Moduls (200) verbunden ist.

2. Verfahren nach Anspruch 1, **dadurch gekennzeichnet, dass** die Analyse eine mikrobiologische Analyse ist.

3. Verfahren nach einem der Ansprüche 1 oder 2, **dadurch gekennzeichnet, dass** der flexible Behälter (100) eine Öffnung (101) aufweist;
das Modul (200) ein Verbindungsstück (201) aufweist, das dazu geeignet und bestimmt ist, mit dem Verbindungsstück des flexiblen Behälters (106) verbunden zu werden.

4. Verfahren nach Anspruch 3, **dadurch gekennzeichnet, dass** eine Änderung der Formung des flexiblen Behälters (100) nach seinem Verschluss und vor Schritt d) durchgeführt wird, so dass das homogenisierte Gemisch in Kontakt mit dem Verbindungsstück (106) ist, das dazu bestimmt ist, mit dem Verbindungsstück (201) des Moduls (200) verbunden zu werden.

5. Verfahren nach einem der Ansprüche 1 bis 3, **dadurch gekennzeichnet, dass** das Analysesystem Detektionsmittel (202) umfasst, die den selektiven Nachweis wenigstens eines Zielanalyten, gewählt aus Bakterien und Proteinen, ermöglichen.

6. Verfahren nach Anspruch 5, **dadurch gekennzeichnet, dass** die Detektionsmittel (202) spezifische optische oder elektrochemische Biosensoren sind, die durch spezifische Liganden funktionalisiert sind, vorzugsweise gewählt aus Antikörpern, Aptameren und Phagenproteinen, Oligosacchariden oder kleinen organischen Molekülen.

7. Verfahren nach einem der Ansprüche 1 bis 6, **dadurch gekennzeichnet, dass** die Probe fest oder halbfest ist.

8. Analyse-Kit, umfassend:
- wenigstens einen flexiblen Behälter (100), umfassend einen Homogenisierungsbereich (105), eine Öffnung (101) und ein Verbindungsstück (106), wobei das Verbindungsstück außerhalb des Homogenisierungsbereichs (105) angeordnet ist;
- ein Modul (200), das ein Analysesystem oder einen Teil davon umfasst, ein Verbindungsstück (201), das dazu angepasst und bestimmt ist, mit dem Verbindungsstück des flexiblen Behälters (106) verbunden zu werden, wobei das Verbindungsstück (106) einen abgedichteten Zustand aufweist, wenn es nicht verbunden ist, und einen offenen Zustand, der den Durchlass der homogenisierten Mischung erlaubt, wenn es mit dem Verbindungsstück (201) des Moduls (200) verbunden ist.

9. Kit nach Anspruch 8, **dadurch gekennzeichnet, dass** das Analysesystem Detektionsmittel (202) umfasst, die den selektiven Nachweis wenigstens eines Zielanalyten ermöglichen, gewählt aus Bakterien sowie Proteinen, Polysacchariden, Fettsäuren und Nukleinsäuren, aus denen diese bestehen oder die diese emittieren.

10. Kit nach Anspruch 9, **dadurch gekennzeichnet, dass** die Detektionsmittel (202) spezifische optische oder elektrochemische Biosensoren sind, die durch spezifische Liganden funktionalisiert sind, vorzugsweise gewählt aus Antikörpern, Aptameren und Phagenproteinen, Oligosacchariden oder kleinen organischen Molekülen.

## Claims

1. A method for analysing a sample comprising:
a) the introduction of the sample and a culture medium in a flexible container (100);
b) the homogenisation of the sample and the culture medium so as to obtain a homogenised mixture;
c) the enrichment of the homogenised mixture; and
d) the analysis of the homogenised mixture by an analysis system,
**characterised in that**
steps a) to c) are performed in a single container (100);
the flexible container (100) is closed after of one of steps a) or b);
a module (200) comprising the analysis system is connected to the flexible container (100) at the start of, during or at the end of step c);
steps c) and d) and the connection of the module (200) to the flexible container (100) are carried out without requiring the reopening of the container;
the flexible container (100) comprising a homogenisation zone (105) and a socket (106) which is located outside the homogenisation zone (105), said socket (106) being sealed when it is not connected and allows the homogenised mixture to pass when it is connected to a socket (201) of the module (200).

2. The method according to claim 1, **characterised in that** the analysis is a microbiological analysis.

3. The method according to one of claims 1 or 2, **characterised in that**
the flexible container (100) comprises an opening (101);
the module (200) comprises a socket (201) adapted and intended to be connected with the socket of the flexible container (106).

4. The method according to claim 3, **characterised in that** a change in conformation of the flexible container (100) is carried out after the closure thereof and before step d) so that the homogenised mixture is in contact with the socket (106) which is intended to be connected with the socket (201) of the module (200).

5. The method according to one of claims 1 to 3, **characterised in that** the analysis system comprises detection means (202) allowing the selective detection of at least one target analyte selected from bacteria and proteins.

6. The method according to claim 5, **characterised in that** the detection means (202) are specific optical or electrochemical biosensors functionalised by specific ligands, preferably selected from the antibodies, the aptamers and the phage proteins, oligosaccharides or small organic molecules.

7. The method according to one of claims 1 to 6, **characterised in that** the sample is solid or semi-solid.

8. An analysis kit comprising:
- at least one flexible container (100) comprising a homogenisation zone (105), an opening (101) and a socket (106), said socket being located outside the homogenisation zone (105);
- a module (200) comprising an analysis system or a portion thereof, a socket (201) which is adapted and intended to be connected with the socket of the flexible container (106), said socket (106) having a sealed state when it is not connected and an on state, allowing the homogenised mixture to pass, once connected to the socket (201) of the module (200).

9. The kit according to claim 8, **characterised in that** the analysis system comprises detection means (202) allowing the selective detection of at least one target analyte selected from bacteria as well as proteins, polysaccharides, fatty acids and nucleic acids which compose them or which they emit.

10. The kit according to claim 9, **characterised in that** the detection means (202) are specific optical or electrochemical biosensors functionalised by specific ligands, preferably selected from the antibodies, the aptamers and the phage proteins, oligosaccharides or small organic molecules.
